# EUROPÄISCHE PATENTSCHRIFT

(11) **EP 2 864 280 B1**
(45) Veröffentlichungstag und Bekanntmachung des Hinweises auf die Patenterteilung: **10.11.2021**
(21) Anmeldenummer: 13730879.7
(22) Anmeldetag: 20.06.2013
(51) Int. Cl.: C07C 45/72, B01J 23/10, B01J 37/08

(54) **HERSTELLUNGSVERFAHREN EINES TRÄGERKATALYSATORS**
PREPARATION PROCESS OF A SUPPORTED CATALYST
PROCÉDÉ DE PRÉPARATION D'UN CATALYSEUR SUPPORTÉ

(30) Priorität: 22.06.2012 DE 102012012784; 22.06.2012 DE 102012012785
(43) Veröffentlichungstag der Anmeldung: 29.04.2015
(73) Patentinhaber: Symrise AG, 37603 Holzminden (DE)
(72) Erfinder: HÖLDERICH, Wolfgang, 67227 Frankenthal (DE); RITZERFELD, Verena, 52072 Aachen (DE)
(74) Vertreter: Fabry, Bernd
(86) Internationale Anmeldenummer: PCT/EP2013/062872
(87) Internationale Veröffentlichungsnummer: WO 2013/190038

(56) Entgegenhaltungen:
- EP-A1- 2 409 961
- EP-A2- 1 164 119
- WO-A1-03/047748
- DE-A1-102009 006 777
- US-A1- 2003 176 752
- US-A1- 2007 083 066
- RUSSBUELDT B M E ET AL: "New rare earth oxide catalysts for the transesterification of triglycerides with methanol resulting in biodiesel and pure glycerol", JOURNAL OF CATALYSIS, ACADEMIC PRESS, DULUTH, MN, US, Bd. 271, Nr. 2, 4. Mai 2010 (2010-05-04), Seiten 290-304, XP027021150, ISSN: 0021-9517 [gefunden am 2010-03-20]
- HOELDERICH WOLFGANG F ET AL: "Preparation of a raspberry ketone precursor in the presence of rare earth oxide catalysts", APPLIED CATALYSIS A: GENERAL, ELSEVIER, AMSTERDAM, NL, vol. 504, 10 April 2015 (2015-04-10), pages 654-663, XP029278677, ISSN: 0926-860X, DOI: 10.1016/J.APCATA.2015.03.033
- HATTORI H: "Heterogeneous Basic Catalysis", CHEMICAL REVIEWS, AMERICAN CHEMICAL SOCIETY, US, vol. 95, no. 3, 1 January 1995 (1995-01-01), pages 537-558, XP002180522, ISSN: 0009-2665, DOI: 10.1021/CR00035A005
- S. J. Tauster ET AL: "Strong metal-support interactions. Group 8 noble metals supported on titanium dioxide", Journal of the American Chemical Society, vol. 100, no. 1, 1 January 1978 (1978-01-01), pages 170-175, XP055234316, US ISSN: 0002-7863, DOI: 10.1021/ja00469a029
- MERCERA P D L ET AL: "ZIRCONIA AS A SUPPORT FOR CATALYSTS. ÖINFLUENCE OF ADDITIVES ON THE THERMAL STABILITY OF THE POROUS TEXTURE OF MONOCLINIC ZIRCONIA", APPLIED CATALYSIS, AMSTERDAM, NL, vol. 71, 1 January 1991 (1991-01-01), pages 363-391, XP000472742, ISSN: 0166-9834, DOI: 10.1016/0166-9834(91)85092-A
- PENGPANICH S ET AL: "Catalytic oxidation of methane over CeO"2-ZrO"2 mixed oxide solid solution catalysts prepared via urea hydrolysis", APPLIED CATALYSIS A: GENERAL, ELSEVIER, AMSTERDAM, NL, vol. 234, no. 1-2, 8 August 2002 (2002-08-08), pages 221-233, XP004370589, ISSN: 0926-860X, DOI: 10.1016/S0926-860X(02)00230-2
- GONZALO-JUAN I ET AL: "Influence of the urea content on the YSZ hydrothermal synthesis under dilute conditions and its role as dispersant agent in the post-reaction medium", JOURNAL OF THE EUROPEAN CERAMIC SOCIETY, ELSEVIER SCIENCE PUBLISHERS, BARKING, ESSEX, GB, vol. 29, no. 15, 1 December 2009 (2009-12-01), pages 3185-3195, XP026499489, ISSN: 0955-2219, DOI: 10.1016/J.JEURCERAMSOC.2009.04.041 [retrieved on 2009-07-08]

## Beschreibung

### GEBIET DER ERFINDUNG

Die Erfindung befindet sich auf dem Gebiet der präparativen organischen Chemie und betrifft ein Verfahren zur Herstellung eines Trägerkatalysators für die Aldolkondensation und für die Kondensation von Carbonylverbindungen.

### TECHNOLOGISCHER HINTERGRUND

Aldolkondensationen sind in der Organischen Chemie eine der am häufigsten angewandten Synthesestrategien zur Knüpfung von C-C-Bindungen. Durch Umsetzung von Ketonen mit Aldehyden bei höheren Temperaturen in Gegenwart geeigneter Katalysatoren entstehen nach OH⁻ Eliminierung *α*-, *β*-ungesättigte Carbonylverbindungen, welche oft wichtige Vorstufen für Feinchemikalien wie Duftstoffe, Aromen, Vitaminen und Pharmaprodukten darstellen. Dies wird am Beispiel des 4-(4-Hydroxyphenyl)-3-buten-2-on veranschaulicht, das durch Aldolkondensation von Benzaldehyd und Aceton erhalten wird. Dies ist ein wichtiges Intermediat für die Synthese des Duft- und Aromastoffes Rheosmin; durch Hydrierung kann es in Rheosmin, 4-(4-Hydroxy-phenyl)-3-butan-2-on, dem sog. Himbeerketon überführt werden. Im Jahre 1957 entdeckten Schinz und Seidel diese Verbindung als Hauptinhaltsstoff und Geruchsträger von roten Himbeeren, welcher einen äußerst geringen Geruchs- und Geschmacksschwellenwert aufweist. Da sein Verzehr als generell unbedenklich eingestuft wird, kann es zur Aromatisierung von Speisen und Getränken jeglicher Art eingesetzt werden. Natürliches Himbeerketon ist einer der teuersten Aromastoffe, die in der Lebensmittelindustrie verwendet werden. Neben seinem Vorkommen in Himbeeren tritt es auch als Inhaltsstoff anderer Früchte wie Pfirsichen, Weintrauben und Äpfeln auf. In der Parfümerie und Kosmetik wird es daher Duftstoffkompositionen, die eine fruchtige Note aufweisen sollen, zugefügt. Darüber hinaus findet das Himbeerketon ebenfalls Verwendung als Insektenlockstoff.

Obwohl es eine wesentliche Rolle im Aroma der Himbeere übernimmt, ist eine Extraktion des Rheosmins direkt aus der Frucht nicht wirtschaftlich, da es in der Regel in nur sehr geringen Konzentrationen von etwa 1 mg pro kg Himbeeren enthalten ist. Um den großen Bedarf an Himbeerketon zu stillen, wurden einfache chemische Syntheseverfahren entwickelt. Das häufigste angewandte Syntheseverfahren für das 4-(4-Hydroxyphenyl)-3-buten-2-on ist die basenkatalysierte Aldolkondensation von 4-Hydroxybenzaldehyd mit Aceton. Durch nachfolgende Hydrierung wird das Himbeerketon gewonnen. Zunächst wurde die Aldolkondensation von 4-Hydroxybenzaldehyd und Aceton vermehrt homogen katalysiert.

### STAND DER TECHNIK

So wird beispielsweise in der DE 2417357 **OS** ein Verfahren zur Herstellung von Aldol-kondensaten beschrieben, in dem Carbonylverbindungen der allgemeinen Formel RCH₂COR¹, in der R und R¹ Wasserstoffe, Alkyl-oder Arylreste sind bzw. R und R¹ zu einem Ring verknüpft sein können, mit sich selbst oder anderen Carbonylverbindungen in Gegenwart von Homogenkatalysatoren umgesetzt. Als solche Katalysatoren werden Dialkylborylcarboxylate der allgemeinen Formel R²₂BOCOR³, in der R³ =C₁-C₆ Kohlenwasserstoffe und R² =C₁-C₄ Kohlenwasserstoffe sind, verwendet. Obwohl bei diesem Verfahren hohe Reaktionsgeschwindigkeiten erreicht und teilweise Ausbeuten bis zu 99 % erzielt werden, zeigen die in diesem Patent vorgestellten Beispiele jedoch, dass eine sehr hohe Katalysatormenge und teuere Bororganyle erforderlich sind. Darüber hinaus bringt die homogene Verfahrensweise einige typische Nachteile mit sich, wie z. B. die schlechte Abtrennung des Katalysators vom Reaktionsgemisch und eine anfallende Salzfracht durch den Aufarbeitungsprozess.

Ein mittlerweile weitverbreiteter Weg Aldolkondensate wie das 4-(4-Hydroxyphenyl)-3-buten-2-on herzustellen, stellt die Biokatalyse dar. Allgemein betrachtet gibt es hierbei zwei verschiedene Ansätze, nämlich die *in vitro*-Synthese mit isolierten Enzymen und die *in vivo*-Synthese mit ganzen Zellen. Diese enzymatischen Prozesse haben den Nachteil, dass nur niedrige Raumzeitausbeuten erzielt werden und die Entsorgung der enzymatischen Abfälle z.B. durch Verbrennung recht aufwendig ist.

Aus dem Stand der Technik ist auch der Einsatz von heterogenen Katalysatoren speziell unter Einbeziehung von Seltenen Erden für die Aldolkondensation grundsätzlich bekannt:

Die Veröffentlichung J CATALYSIS 271, S. 290ff (2010**)** betrifft Seltene Erdenoxid-Katalysatoren für die Umesterung von Triglyceriden mit Methanol für die Herstellung von Biodiesel. Im einzigen relevanten Abschnitt 2.2.3. wird die Herstellung eines Katalysator beschrieben, bei dem im ersten Schritt Zirkonhydroxid mit 8 bis 10 Gew.-% Lanthanoxid oder Yttriumoxid vermischt und dann bei 700 C über insgesamt 12 h kalziniert wird. Dementgegen werden im Verfahren gemäß vorliegender Erfindung dem Zirkonoxid keine Oxide der Seltenen Erden zugegeben, sondern Nitrate, die anschließend als Hydroxide oder Carbonate ausgefällt und auf dem Zirkonoxid abgelagert werden, bevor im nachfolgenden Schritt die Kalzinierung erfolgt, bei dem dann aus den Hydroxiden bzw. Carbonaten die Oxide gebildet werden. Anschließend wird das Zwischenprodukt in gleicher Weise mit Lösungen von SE-Salzen imprägniert, getrocknet und abermals kalziniert.

Die Veröffentlichung APPL. CAT. 71, S. 363-391 (1991**)** stellt einen Übersichtsartikel zu Zirkondioxid als Träger für Katalysatoren dar. Konkret geht es um die thermische Stabilität von monoklinem ZrO₂. Wie auf den Seiten 366/367 beschrieben, wurde ZrO₂ zunächst alleine bei 400 C kalziniert, anschließend mit SE-Nitratsalz-Lösungen imprägniert und dann bei 400 C abermals kalziniert. Damit unterscheidet sich das Herstellverfahren in wesentlichen Punkten ganz entscheidend von der vorliegenden Erfindung. Insbesondere befinden sich die SE nur in den Poren, da der Vorgang der Präzipitation fehlt und die Kalzinierung erfolgt bei deutlich niedrigeren Temperaturen. Abgesehen davon wird ausschließlich monoklines ZrO₂ eingesetzt, während erfindungsgemäß wenigstens teilweise tetragonal-stabilisiertes ZrO₂ zum Einsatz kommt.

Die Veröffentlichung APPL. CAT. A: General, 234, S.221-233 (2002**)** beschäftigt sich mit Zirkonoxid, das mit Yttrium dotiert ist und seine katalytischen Eigenschaften. Beschreiben wird die Behandlung einer Lösung von Zirkon- und Yttriumsalzen nach Zugabe von Harnstoff. Die resultierenden Pulver wurden bei Temperaturen unterhalb von 200 C behandelt. Weitere Verfahrensschritte sind weder genannt noch in Betracht gezogen. Daher ist auch diese Schrift nicht relevant.

In der DE 2615308 **AS** wird ein Verfahren zur Herstellung höherer Ketone durch Kondensation eines Aldehyds mit einem in flüssiger Phase beschrieben. Dabei wird ein Katalysatorsystem eingesetzt, das sowohl kondensierende als auch hydrierende Eigenschaften besitzt. Während zur Katalyse der Kondensation Oxide der Seltenen Erden bevorzugt CeO₂, Pr₂O₃ und Nd₂O₃ auf Trägern wie Aluminiumoxid, Aktivkohle, Bimsstein, Silikagel, Kieselgur und Zeolithe, unter denen Aluminiumoxid und Aktivkohle bevorzugt sind, eingesetzt werden, werden Edelmetalle wie Palladium auf Aktivkohle für die anschließende Hydrierung verwendet.

Gegenstand der EP 0013385 A1 (BASF) ist ein Verfahren zur Herstellung von aliphatischen Carbonylverbindungen durch Aldolkondensation, bei dem heterogene Katalysatoren eingesetzt werden, die Mischungen von Metallen der VIII Hauptgruppe und Oxiden oder Salzen der Seltenen Erden darstellen. Die Beispiele offenbaren konkret Katalysatoren, bei denen als Trägermaterial Aluminiumoxid, Siliziumdioxid oder Pd/Aktivkohle und als aktive Komponenten Palladium in Kombination mit den Oxiden von Praseodym, Cer und Lanthan eingesetzt werden. Die Oxide der übrigen Seltenen Erden werden in der Beschreibung ebenfalls als geeignet offenbart.

Die EP 0623382 A1 (Exxon) offenbart Katalysatoren für die Herstellung von verzweigten Alkoholen via Aldolkondensation, wobei der Vorteil darin liegt, bei relativ niedrigem Druck arbeiten zu können. Dabei kommen Fällungskatalysatoren zum Einsatz, die ein Oxid eines Metalls aus der Gruppe IIA und ein Oxid eines Metalls aus der Gruppe der Seltenen Erden enthält und üblicherweise auf einem Cu-Träger niedergeschlagen werden. Offenbart werden in den Beispielen Mischkatalysatoren, die als IIA-Metall Magnesium und als Seltene Erden Cer, oder Yttrium aufweisen.

Gegenstand der EP 220099 B1 (Universität Amsterdam) sind spezielle Komplexverbindungen mit Schwefel-Stickstoff-Phosphor-Liganden, die z.B. an ein Lanthanidenmetall als Zentralmetall gebunden sein können. Die Komplexe eignen sich u.a. als Katalysatoren für die Aldol-Kondensation

Die beiden internationalen Patentanmeldungen WO 2003 047747 A1 und WO 2003 047748 A1 (BASF) beschreiben die Herstellung von Pseudoionon durch Aldolkondensation von Citral und Aceton in einer speziellen Kolonne, wobei als Katalysatoren Oxide von Lanthan, Yttrium und insbesondere Praseodym eingesetzt werden

Gegenstand der WO 2012/022562 A1 (DSM) ist die Herstellung von Pseudoionon durch Aldolkondensation. Beansprucht wird die Verwendung von reinem Lanthanoxid in der Aldolkondensation von Citral und Aceton. In Tabelle 2 finden sich auch Beispiele für Neodym- und Praseodymoxid.

Aus der US 5,559,275 ist ein Verfahren zur Aldolkondensation von niederen aliphatischen Alkoholen bekannt, wobei Katalysatoren eingesetzt werden, die als Träger beispielsweise Titan- oder Zirkonoxid und als aktive Spezies Metalle der VIII Hauptgruppe gegebenenfalls in Kombination mit Alkali- oder Erdalkalien enthalten. Der Träger kann des Weiteren auch Ceroxid.

Schließlich werden in der chinesische Patentanmeldung CN 102600827 A Mischkatalysatoren für die Aldolkondensation vorgeschlagen, die man erhält, indem man Alkali- oder Erdalkalimetall und Seltene Erden auf einen Träger aufbringt und dann gemeinsam kalziniert.

### AUFGABE DER ERFINDUNG

Den Verfahren aus dem Stand der Technik ist der Nachteil gemein, dass sie bei eher durchschnittlichen Ausbeuten nur unbefriedigende Selektivitäten aufweisen. Werden homogene bzw. enzymatische Katalysatoren eingesetzt, können diese nur mit hohem Aufwand vom Wertprodukt abgetrennt werden, bei heterogenen Katalysatoren besteht der Nachteil darin, dass sie unbefriedigende Standzeiten aufweisen und sich nur aufwendig regenerieren lassen, wobei nicht selten das ursprüngliche Leistungsvermögen nicht wieder erreicht wird.

Die komplexe Aufgabe der vorliegenden Erfindung hat demnach darin bestanden, neue heterogene Katalysatoren für die Kondensation von Carbonylverbindungen, speziell für die Aldolkondensation zur Verfügung zu stellen, die die geschilderten Nachteile des Stands der Technik überwinden. Insbesondere sollten die Katalysatoren bei mindestens gleichwertigen Ausbeuten eine deutlich verbesserte Selektivität, vorzugsweise über 95 % aufweisen und die Kondensation unterschiedlichster Carbonylverbindungen in kurzen Zeiten und bei moderaten Reaktionsbedingungen katalysieren. Die Katalysatoren sollten über eine hohe Standzeit verfügen, sich leicht abtrennen und regenerieren lassen und anschließend wieder ohne Leistungseinbuße einsetzbar sein.

### BESCHREIBUNG DER ERFINDUNG

Gegenstand der Erfindung ist ein Verfahren zur Herstellung eines Trägerkatalysators mit teilweise oder vollständig tetragonal stabilisiertem Zirkondioxid, bei dem man
(a) Zirkonhydroxid mit 10% Massenprozent der Nitrate der Seltenen Erdmetalle versetzt,
(b) die Nitrate der Seltenen Erdmetalle als Hydroxide mit Hilfe von Harnstoff langsam über 18 Stunden ausfällt,
(c) die Mischung mindestens 6 h unter Rückfluss erhitzt, abgekühlt, filtriert und trocknet,
(d) den resultierende Feststoff bei 700 bis 1.000 °C kalziniert,
(e) das so erhaltene Trägermaterial mit einer wässrigen Lösung eines reinen Salzes der Seltenen Erdmetalle imprägniert, und
(f) das Umsetzungsprodukt zunächst von Wasser befreit und dann bei Temperaturen oberhalb von 500 °C kalziniert.

Überraschenderweise wurde gefunden, dass sich mit Hilfe des neuen Verfahrens Trägerkatalysatoren für die Aldolkondensation herstellen lassen, mit denen sich Ausbeute und Selektivität gegenüber Katalysatoren des Stands der Technik noch einmal deutlich verbessern lassen. Insbesondere werden regelmäßig Selektivitäten oberhalb von 95 %, typisch zwischen 99 und 100 % erzielt. Die Katalysatoren lassen sich einfach herstellen, weisen eine hohe Standzeit auf und können ebenso einfach vom Reaktionsprodukt abgetrennt werden. Sie lassen sich leicht regenerieren und anschließend ohne Leistungseinbuße wiederverwenden.

### Trägerkatalysatoren

Der Begriff Seltene Erden umfasst hierbei die Elemente Yttrium, Lanthan, Cer, Praseodym, Neodym, Samarium und Ytterbium und ein beliebiges Gemisch dieser Lanthanoide. Der erfindungsgemäß hergestellte Katalysator stellt einen Trägerkatalysator dar, der aus einem Oxid der Seltenen Erden insbesondere Lanthanoxid und ZrO₂ als Träger besteht. Die mittlere Oberflächen des Trägers (bestimmt nach BET) liegen vorzugsweise im Bereich von etwa 10 bis etwa 250 und insbesondere etwa 50 bis etwa 200 m²/g. Die Träger können aus teilweise bzw. vollständig tetragonalem ZrO₂ bestehen. Bevorzugte Mengenverhältnisse liegen im Bereich von etwa 0,2 bis etwa 30 Gew.-%, insbesondere etwa 0,5 bis etwa 20 Gew.-% und besonders bevorzugt etwa 1 bis etwa 5 Gew.-% an Selten Erdmetalloxid.

Die katalytisch aktive Komponente kann durch Fällung mit einem geeigneten Fällungsmittel au den Träger aufgebracht werden. Hierzu wird eine Suspension des Trägermaterials und des in Wasser gelösten Nitrates der Seltenen Erden mit einem geeigneten Fällungsmittel, beispielsweise einer Carbonsäure wie der Oxalsäure versetzt. Zum anderen können die Träger mit einer wässrigen Lösung der wasserlöslichen reinen Salze der Seltenen Erden, bevorzugt den Nitraten der Seltenen Erden, imprägniert werden. Bevorzugt lassen sich als Träger tetragonal stabilisiertes Zirkondioxid ZrO₂ (z.B. von Mel Chemicals, Manchester) und Titandioxid (z.B. von P25 Evonik Industries AG, Hanau) mit La(NO₃)₃ in der beschriebenen Weise imprägniert, von Wasser befreit und dann bei Temperaturen oberhalb von 500 °C, vorzugsweise 700 bis 100 °C kalziniert. Wesentliche Bestandteile des einsatzbereiten Zirkondioxidkatalysators bilden dann tetragonales ZrO₂, La₂O₃ und die Mischphase La₂Zr₂O₇ in beliebigen Mengenverhältnissen. Die Mischphase La₂Zr₂O₇ weist Pyrochlorstruktur auf.

Die Katalysatoren werden vorzugsweise durch Herstellen von tetragonalem ZrO₂ und Imprägnieren von tetragonalem ZrO₂ mit den entsprechenden Nitraten der Seltenen Erden erhalten.

### Aldolkondensation

Die erfindungsgemäß hergestellten Trägerkatalysatoren können in Verfahren zur Herstellung von Aldolkondensaten durch Umsetzung einer ersten Carbonylverbindung mit sich selbst oder einer zweiten Carbonylverbindung eingesetzt werden. Insbesondere können die ersten Carbonylverbindungen Ketoverbindungen der Formel (I) darstellen,

RCH₂COR^{Í} (I)

wobei R = H, lineare oder verzweigte Alkyl-, Cycloalkyl-, Aryl- und Aralkyl-Gruppe aber auch heterocyclische Gruppe mit N, O oder S als Heteroatome und heteroaromatische Gruppe mit N, O oder S als Heteroatome bedeutet und wobei R^{I} für H, lineare oder verzweigte Alkyl-, Cycloalkyl- Aryl- und Aralkyl- Gruppe, aber auch für heterocyclische Gruppe mit N, O oder S als Heteroatome und heteroaromatische Gruppe mit N, O oder S als Heteroatome steht und hierbei die aromatischen Ringe mono - oder mehrfach mit linearen oder verzweigten Alkyl- , Cycloalkyl-, Hydroxy-, Alkoxy-, Phenoxy-, Carboxy-, Amino- und Halogenresten insbesondere F und CF₃ substituiert sein können.

Die zweiten Carbonylverbindungen können der Formel (II) folgen,

R^{II}OR^{III} (II)

wobei R" und R^{III} gleich oder verschieden sind und R^{II} und R^{III} = H, lineare oder verzweigte Alkyl-, Cycloalkyl-, Aryl- und Aralkyl-Gruppen, aber auch heterocyclische Gruppen mit N, O oder S als Heteroatome und heteroaromatische Gruppen mit N, O oder S als Heteroatome bedeuten, wobei die aromatischen Ringe mono - oder mehr- fach mit linearen oder verzweigten Alkyl-, Cycloalkyl-, Hydroxy-, Alkoxy-, Phenoxy-, Carboxy-, Amino- und Halogenresten insbesondere F und CF₃ substituiert sein können und die einzelnen Reste R, R^{I}, R", R^{III} dabei auch untereinander zu Ringen mit einer C Zahl von 4- 12, vorzugsweise 5 und 6 verknüpft sein können einschließlich Ringe mit N, O oder S als Heteroatome.

Beispielsweise können folgende Einsatzstoffe zu wertvollen Aldolkondensaten umgesetzt werden:
- 4-Hydroxybenzaldehyd ( 4-HBA ) + Aceton zu 4-(4-Hydroxyphenyl)-3-buten-2-on, das für die Herstellung des Himbeerketons genutzt wird.
- Benzaldehyd + Acetaldehyd zu Zimtaldehyd, ein Duftstoff, der stark nach Zimt riecht.
- Acetaldehyd + Acetaldehyd zu Crotonaldehyd, der ein bedeutende Rolle in der Vitaminproduktion spielt, der sich leicht zur Crotonsäure oxidieren und zum Crotylalkohol reduzieren lässt.
- Vanillin + Aceton zu Vanillylaceton = Zingerone, das dem Aromastoff Ingwer / Ginger entspricht.
- Methylcyclohexanon + Aceton zu Pulegon (1-methyl-4-isopropyliden-3-cyclohexanon) das als Ausgangsstoff in der Synthese von Parfümölen, als Inhaltsstoff in Insektenrepellants oder zur Synthese von Menthol dient.
- 5-Hydroxymethylfurfural ( 5-HMF ) + Aceton zu 5-Hydroxymethylfurylbutenon, welches als C9-Baustein nach entsprechender Hydrierung zum entsprechenden Alkan als Treibstoffkomponente eingesetzt werden kann.

Die beschriebenen Aldolkondensationen können in an sich bekannter Weise in flüssiger Phase durchgeführt werden. Im Sinne der vorliegenden Erfindung ist es jedoch ebenfalls möglich, die Reaktionen in der Gasphase oder unter bestimmten Bedingungen in der superkritischen Phase durchzuführen.

Die Reaktion wird in der Flüssigphase zweckmässigerweise in einem Temperaturbereich von etwa 100 bis etwa 250 °C, vorzugsweise in einem Bereich von etwa 160 bis etwa 200 °C und unter Normaldruck oder Drucken von etwa 1 bis etwa 40 bar, vorzugsweise Normaldruck bis etwa 25 bar durchgeführt. Bei niedrigeren Temperaturen wird die Ausbeute so gering, dass das Verfahren für eine kommerzielle Anwendung meistens nicht mehr interessant ist. Werden unterschiedliche Carbonylverbindungen kondensiert, empfiehlt es sich mit einem Überschuss vorzugsweise der billigeren Komponente zu arbeiten, beispielsweise im molaren Verhältnis (etwa 2 bis etwa 18):1, vorzugsweise (etwa 6 bis etwa 14):1. Das molare Verhältnis von Katalysator zu Carbonylverbindung liegt zweckmässigerweise im Bereich von etwa 1:1000 bis etwa 1:10 und vorzugsweise etwa 1:20 bis etwa 1:100.

Das Verfahren ist, wie die Beispiele belegen, für diskontinuierlichen Betrieb geeignet. Die Flüssigphasenreaktion kann batchweise in einem Autoklaven oder einem Rührkessel stattfinden, wobei das Reaktionsgemisch unter Rühren auf eine bestimmte Temperatur erwärmt und für die Dauer der Reaktion bei dieser gehalten wird. Nach Ablauf der Reaktion wird das Produktgemisch gequencht, d.h. beispielsweise im Eisbad abgekühlt. Nach der Reaktion wird der Katalysator durch Filtration abgetrennt. Die Analyse der Produktmischung erfolgt über die Gaschromatographie. Die Quantifizierung erfolgt über GC mit einem internen Standard.

Das Verfahren ist, wie die Beispiele belegen, auch für den kontinuierlichen Betrieb geeignet und kann beispielsweise in einer Rührkesselkaskade oder in einem Schlaufenreaktor oder in einem Tricklebett Reaktor oder einem Plattenreaktor durchgeführt werden,

Das Verfahren kann auch in der Gasphase in einem Festbettreaktor oder Plattenreaktor durchgeführt werden. Dabei wird bei Temperaturen zwischen etwa 180 °C und etwa 400°C, bevorzugt etwa 250 bis etwa 320°C , bei Normaldruck oder bei Drücken zwischen etwa 1 und etwa 10 bar, bevorzugt bei etwa 3 bis etwa 6 bar und bei Verhältnissen der Reaktionskomponenten im Bereich (etwa 18 bis etwa 2) : 1 Mol/Mol, vorzugsweise im Bereich (etwa 14 bis etwa 6) : 1 Mol/Mol gearbeitet.

Auch ist es unter Einstellung gewisser Verfahrensparameter möglich, die Reaktionen unter superkritischen Bedingungen durchzuführen. Hierdurch kann man einer Katalysatordesaktivierung durch Abscheidung von höher molekularen Verbindungen oder Koks vorbeugen.

Der Katalysator kann mehrfach verwendet werden, wobei allerdings seine Aktivität durch Wasseraufnahme abnehmen. Es sollte daher in regelmässigen Abständen, die leicht empirisch bestimmt werden können, regeneriert, z.B. bei 500°C in Luftatmosphäre rekalziniert werden.

Nach der Reaktion wird der Katalysator durch Filtration abgetrennt. Die Analyse der Produktmischung erfolgt über die Gaschromatographie. Die Quantifizierung erfolgt über einen internen Standard.

### BEISPIELE

### Verwendete Chemikalien

Es wurden Lanthanoxid La₂O₃, Neodymoxid Nd₂O₃, Praseodymoxid Pr₂O₃ und Samariumoxid Sm₂O₃, Yttriumoxid Y₂O₃, Ytterbiumoxid Yb₂O₃ und Cernitrat Ce(NO₃)₃ mit einer Reinheit von 99,9 % von ABCR Karlsruhe bezogen. Die eingesetzte Oxalsäure wurde mit einer Reinheit von 98 % von Sigma Aldrich und der Harnstoff mit einer Reinheit von >99 % von Merck gekauft. Weiterhin wurden 65%ige Salpetersäure HNO₃ der Fa. Merck und demineralisiertes (VE) Wasser verwendet.

Als Katalysatorträgermaterialien kann ZrO₂ (Mel Chemicals, Manchester) eingesetzt werden. Es kann auch Zr(OH)₄ dotiert mit 10 % La₂O₃ XZO 1526/01 als Pulver (Mel Chemicals, Manchester) als Katalysator verwendet werden.

Die Einsatzstoffe für die Aldolkondensationen wurden von Fa. Merck und von Alfa Aesar, Karlsruhe, erhalten.

### Vergleichsbeispiel V1, Beispiele 1 bis 4

Diese Beispiele zeigen die katalytische Wirksamkeit von Trägerkatalysatoren aus Oxiden der Seltenen Erden in der Aldolkondensation von Aceton und 4-Hydroxybenzaldehyd zu 4-(4-Hydroxyphenyl)-3-buten-2-on Die Katalysatoren wurden durch Herstellen von tetragonalem ZrO₂ und Imprägnieren von tetragonalem ZrO₂ mit den entsprechenden Nitraten der Seltenen Erden erhalten.

Um zumindest teilweise oder komplett tetragonal stabilisiertes Zirkondioxid zu erhalten, wurden die Nitrate der Seltenen Erden mit Hilfe von Harnstoff langsam über 18 Stunden verteilt als Hydroxide bzw. Carbonate ausgefällt. Hierzu wurden 26,87 g (0,170 mol) Zirkonhydroxid verwendet und entsprechend mit 10 % Massenprozent der Nitrate der Seltenen Erden versetzt, z. B. 4 g (0,012 mol) Lanthannitrat La(NO₃)₃. Anschließend wurde die sechsfache Stoffmenge Harnstoff als Fällungsreagenz hinzugegeben, im Fall der lanthanhaltigen Katalysatoren 4,4 g (0,078 mol) und die Mischung mit 50 mL VE-Wasser versetzt. Die Reaktionsmischung wurde über Nacht unter Rückfluss erhitzt und mittels eines KPG-Rührers gerührt. Dann wurde die Katalysatorvorstufe filtriert, mit VE-Wasser neutral gewaschen und bei 120 °C 18 Stunden getrocknet. Im nächsten Schritt wurde diese Katalysatorvorstufe bei 700 °C 6 Stunden lang mit einer sechsstündigen Aufheizrampe von Raumtemperatur auf 700 °C kalziniert

Im Folgenden wurden 18 g teilweise tetragonal stabilisiertes Zirkondioxid t-ZrO₂/ 10 %La₂O₃ mit weiteren zehn Massenprozent der Nitrate der Seltenen Erden imprägniert, z. B. 4 g (0,012 mol) Lanthannitrat La(NO₃)₃, bei 120 °C 18 Stunden getrocknet und gut gemörsert. Anschließend wurde bei 700 °C 6 Stunden lang mit einer Aufheizrampe von Raumtemperatur auf 700 °C von 6 Stunden isotherm kalziniert. Auf analoge Weise werden die anderen verwendeten Oxide der Seltenen Erden auf den Träger aufgebracht. Für die Katalyseversuche wurden 23,7 g Aceton, 4,53 g 4-Hydroxybenzaldehyd, molares Verhältnis (Aceton:4-Hydroxybenzaldehyd) 11:1, und 1 g des La₂O₃ auf teilweise tetragonal stabilisiertem Zirkondioxid eingesetzt. Die Reaktion wurde in einem 75 ml Autoklaven für 3 Stunden bei 200 °C und einem autogenem Druck von 25 bar durchgeführt. Die Ergebnisse sind in **Tabelle 1** zusammengefasst.

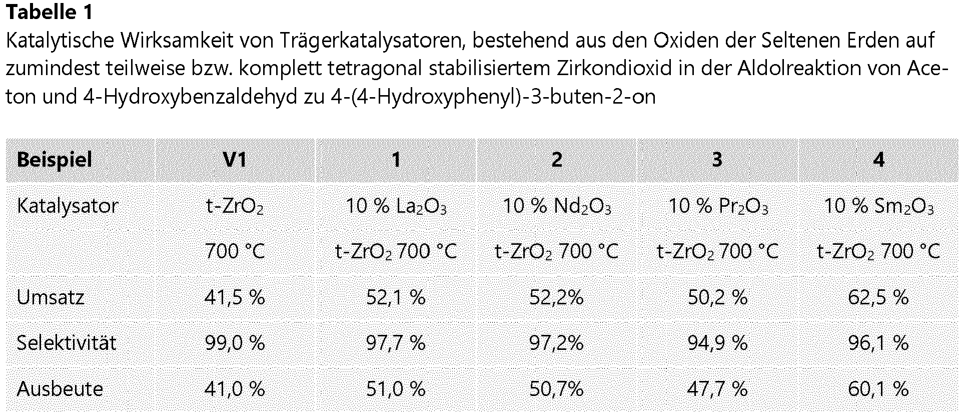

Wie aus dieser Tabelle zu erkennen ist, zeigen die Oxide der Seltenen Erden aufgebracht auf zumindest teilweise oder komplett tetragonal stabilisiertes Zirkondioxid mindestens vergleichbar, in vielen Fällen sogar bessere katalytische Wirksamkeit wie die eingesetzten Vollkatalysatoren.

## Patentansprüche

1. Verfahren zur Herstellung eines Trägerkatalysators mit teilweise oder vollständig tetragonal stabilisiertem Zirkondioxid, bei dem man
(a) Zirkonhydroxid mit 10% Massenprozent der Nitrate der Seltenen Erdmetalle versetzt,
(b) die Nitrate der Seltenen Erdmetalle als Hydroxide mit Hilfe von Harnstoff langsam über 18 Stunden ausfällt,
(c) die Mischung mindestens 6 h unter Rückfluss erhitzt, abgekühlt, filtriert und trocknet,
(d) den resultierende Feststoff bei 700 bis 1.000 °C kalziniert,
(e) das so erhaltene Trägermaterial mit einer wässrigen Lösung eines reinen Salzes der Seltenen Erdmetalle imprägniert, und
(f) das Umsetzungsprodukt zunächst von Wasser befreit und dann bei Temperaturen oberhalb von 500 °C kalziniert.

2. Verfahren nach Anspruch 1, **dadurch gekennzeichnet, dass** man im Schritt (a) Lanthannitrat einsetzt.

3. Verfahren nach Anspruch 1, **dadurch gekennzeichnet, dass** man teilweise tetragonal stabilisiertes Zirkondioxid t-ZrO₂/10 Gew.- % La₂O₃ mit weiteren zehn Massenprozent der Nitrate der Seltenen Erden imprägniert.

4. Verfahren nach Anspruch 3, **dadurch gekennzeichnet, dass** man das imprägnierte Produkt bei 700 °C 6 Stunden lang mit einer Aufheizrampe von Raumtemperatur auf 700 °C von 6 Stunden isotherm kalziniert.

## Claims

1. A process for the preparation of a supported catalyst comprising partially or completely tetragonally stabilized zirconium dioxide, which comprises
(a) adding 10% by mass of rare earth metal nitrates to zirconium hydroxide,
(b) the nitrates of the rare earth metals are precipitated as hydroxides with the aid of urea for a long period of 18 hours,
(c) the mixture is heated under reflux for at least 6 h, cooled, filtered and dried,
(d) alcining the resulting solid at 700 to 1,000 °C,
(e) impregnating the resulting support material with an aqueous solution of a pure salt of the rare earth metals, and
(f) the reaction product is first freed from water and then calcined at temperatures above 500 °C.

2. The process according to claim 1, **characterized in that** lanthanum nitrate is used in step (a).

3. The process according to claim 1, **characterized in that** partially tetragonally stabilized zirconium dioxide t-ZrO₂/10 wt.-% La₂O₃ is impregnated with a further ten mass percent of rare earth nitrates.

4. The process according to claim 3, **characterized in that** the impregnated product is isothermally calcined at 700 °C for 6 hours with a heating ramp from room temperature to 700 °C for 6 hours.

## Revendications

1. Un procédé pour la préparation d'un catalyseur supporté comprenant du dioxyde de zirconium partiellement ou complètement stabilisé en tétragon, qui comprend
(a) en ajoutant 10 % en masse de nitrates de métaux de terres rares à l'hydroxyde de zirconium,
(b) les nitrates des métaux des terres rares sont précipités sous forme d'hydroxydes à l'aide d'urée pendant une longue période de 18 heures,
(c) le mélange est chauffé à reflux pendant au moins 6 heures, refroidi, filtré et séché,
(d) calciner le solide résultant entre 700 et 1 000 °C,
(e) imprégner le matériau de support résultant avec une solution aqueuse d'un sel pur des métaux des terres rares, et
(f) le produit de la réaction est d'abord débarrassé de l'eau, puis calciné à des températures supérieures à 500 °C.

2. Procédé selon la revendication 1, **caractérisé en ce que** le nitrate de lanthane est utilisé dans l'étape (a).

3. Procédé selon la revendication 1, **caractérisé en ce que** le dioxyde de zirconium partiellement stabilisé en tétragon t-ZrO₂/10 % en poids de La₂O₃ est imprégné d'un autre dix pour cent en masse de nitrates de terres rares.

4. Procédé selon la revendication 3, **caractérisé en ce que** le produit imprégné est calciné isothermement à 700 °C pendant 6 heures avec une rampe de chauffage de la température ambiante à 700 °C pendant 6 heures.
